# EUROPÄISCHE PATENTANMELDUNG

(11) **EP 2 281 797 A1**
(43) Veröffentlichungstag der Anmeldung: **09.02.2011**
(21) Anmeldenummer: 09009822.9
(22) Anmeldetag: 29.07.2009
(51) Int. Cl.: C07C 47/06, C07C 45/67

(54) **Verfahren zur selektiven Herstellung von Acetaldehyd aus Acrolein und einem oder mehreren in Wasser gelösten Ammoniumsalzen**

(71) Anmelder: Lonza Ltd., 4052 Basel (CH)
(72) Erfinder: Die Erfindernennung liegt noch nicht vor

(57) **Zusammenfassung**

Die vorliegende Erfindung betrifft ein Verfahren zur selektiven Herstellung von Acetaldehyd, **dadurch gekennzeichnet, dass** Acrolein und ein oder mehrere in Wasser gelöste Ammoniumsalze unter hohen Drücken und Temperaturen von 300-400 °C kontinuierlich umgesetzt werden.

## Beschreibung

Die vorliegende Erfindung betrifft ein Verfahren zur selektiven und kontinuierlichen Herstellung von Acetaldehyd aus Acrolein.

Ein beträchtlicher Anteil an Acetaldehyd wird zur Herstellung von Essigsäureestern verwendet. Beispielsweise wird Ethylacetat in einer Umlagerungsreaktion mit Aluminiumalkoholaten als Katalysator (Claisen-Ti 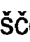 enko-Reaktion) hergestellt. Ein wesentlicher Anteil wird auch mit Formaldehyd zur Produktion von Pentaerythritol, einem Zwischenprodukt bei der Herstellung von Alkylharzen sowie Weichmachern und Emulgatoren, verwendet. Des Weiteren ist Acetaldehyd ein Intermediat bei der Herstellung von Butadien ausgehend vom Acetylen über Acetaldol und dessen Hydrierprodukt 1,3-Butandiol. Die Bildung des Acetaldols, welches zu Crotonaldehyd dehydratisiert werden kann, erfolgt durch Aldol-Addition von Acetaldehyd. Eine zunehmende Bedeutung gewinnt die Umsetzung von Acetaldehyd mit Stickstoffverbindungen zu Pyridin und dessen Derivaten. So wird 5-Ethyl-2-Methylpyridin in einer Flüssigphasenreaktion aus Acetaldehyd und Ammoniak hergestellt. Zugabe von Formaldehyd oder Acrolein führt zur Bildung von Pyridin und Alkylpyridinen. Weitere Anwendung findet Acetaldehyd bei der Produktion von Peressigsäure, bei der Oxidation mit Salpetersäure zu Glyoxal bzw. Glyoxalsäure sowie den Additionsreaktionen mit Blausäure zu Lactonitril, einer Vorstufe des Acrylnitrils und Acetanhydrid zu Ethylidendiacetat, einem Intermediat im Vinylacetat-Prozess [Eck2007].

Nach dem Stand der Technik kann Acrolein u. a. aus der Dehydratisierung von Glycerol, das als "Abfallprodukt" in größeren Mengen bei der Biodiesel-Produktion anfällt, in nah- und überkritischem Wasser unter Zugabe von Säuren [Wat2007] oder Salzen [Ott2006] hergestellt werden. Bei der Umsetzung des gewonnenen Acroleins mit Ammoniumsalzen wird Acetaldehyd in hohen Ausbeuten erhalten.

Es ist bekannt, dass Acetaldehyd bei der Dehydratisierung von Glycerol in überkritischem Wasser bei 500 °C, 34,5 MPa und einer Verweilzeit von 90 s mit einer Ausbeute von 26 % erhalten wird [Ant1985]. Ein radikalischer Mechanismus über Dehydratisierung des Glycerols zum 3-Hydroxypropionaldehyd und homolytischer Spaltung dieses Intermediats zu Acetaldehyd und Formaldehyd wird für die angegebenen Bedingungen angenommen. Ein alternativer Mechanismus über homolytische Spaltung von Acetol kann ausgeschlossen werden. Die Selektivität zu Acetaldehyd ist unter nahkritischen Bedingungen des Wassers bei 360 °C und gleichen Bedingungen geringer. Zugabe von Natriumhydrogensulfat als saurer Katalysator führt lediglich zu einer Erhöhung der Ausbeute an Acrolein, welches vermutlich aus der säurekatalysierten Dehydratisierung des 3-Hydroxypropionaldehyds entsteht.

Es ist weiterhin bekannt, dass Acetaldehyd bei der Dehydratisierung von Glycerol in nahkritischem Wasser zwischen 300 und 350 °C und 34,5 MPa durch Zugabe von 0,005 M Schwefelsäure als Nebenprodukt entsteht [Ant1987]. Die Umsetzung einer 0,5 M Glycerol-Lösung bei 325 °C und einer Verweilzeit von 39 s führt zu einer molaren Ausbeute von Acetaldehyd von lediglich 5 %. Als Reaktionsmechanismus wird eine Retro-Aldol-Reaktion ausgehend von Acrolein über 3-Hydroxypropionaldehyd angenommen, wobei zusätzlich Formaldehyd entsteht, welches unter den angegebenen Bedingungen zu Wasserstoff, Kohlenmonoxid und Kohlendioxid zerfällt. Diese Annahme wurde durch den Einsatz einer äquimolaren Menge an Acetaldehyd und Formaldehyd sowie der Kreuz-Aldol-Reaktion zu Acrolein bestätigt. Die Umsetzung einer verdünnten Acetaldehyd-Lösung unter nicht katalytischen bzw. alkalischen Bedingungen führt zur hauptsächlichen Bildung von Crotonaldehyd. Des Weiteren kann Acetaldehyd als Hauptprodukt durch säurekatalysierte Dehydratisierung aus Ethylenglykol gewonnen werden. Die Dehydratisierung einer 0,5 M Ethylenglykol-Lösung bei 385 °C, 34,5 MPa und einer Verweilzeit von 29 s führt zu einer molaren Ausbeute von Acetaldehyd von 40 %. Nachteilig ist die korrosive Eigenschaft der Schwefelsäure vor allem in nahkritischem Wasser.

Weitere Untersuchungen zur Dehydratisierung von Polyolen unter überkritischen Bedingungen des Wassers (385 °C, 34 MPa, 20-45 s Verweilzeit) sind ebenso bekannt [Ram1987]. Die säurekatalysierte Umsetzung einer 0,5 M Ethylenglykol-Lösung führt zu einer maximalen Ausbeute an Acetaldehyd von 41 % bei 45 s Verweilzeit. Wasserstoff, Kohlenmonoxid, Kohlendioxid und Ethylen können als Nebenprodukte in geringen Mengen identifiziert werden. Bei der Dehydratisierung von Glycerol im nahkritischen Bereich wird Acetaldehyd mit einer maximalen Ausbeute von 12 % bei 350 °C, 25 s Verweilzeit und Zugabe von katalytischen Mengen an Schwefelsäure erhalten. Die Rückreaktion bzw. Kreuz-Aldol-Reaktion von Acetaldehyd mit Formaldehyd zu Acrolein führt zu einer Ausbeute von Acrolein von 22 % bezüglich Formaldehyd. Als weiteres flüssiges Produkt wird Crotonaldehyd nachgewiesen, welches durch Aldol-Reaktion von Acetaldehyd gebildet wird. Die Aldol-Reaktion kann durch Zugabe von Säuren verlangsamt werden.

Die Ergebnisse der Umsetzung von Glycerol ohne Zusatz in nah- und überkritischem Wasser in einem Temperaturbereich von 250-475 °C, bei Drücken von 25, 35 oder 45 MPa, Verweilzeiten von 32-165 s und verschiedenen Anfangskonzentration von Glycerol sind ebenfalls bekannt [Büh2002]. Tiefere Temperaturen sowie höhere Drücke und Verweilzeiten führen zu höheren relativen Selektivitäten bezüglich Acetaldehyd, wobei der maximale Umsatz an Glycerol mit 31 % relativ gering ist. Es werden zwei konkurrierende Reaktionspfade für die Umsetzung von Glycerol beschrieben. Bei höheren Drücken und/oder tieferen Temperaturen werden ionische Reaktionsschritte bevorzugt, wohingegen bei tieferen Drücken und/oder höheren Temperaturen radikalische Umsetzungen erfolgen. Acetaldehyd kann dabei auf beiden Wegen gebildet werden und ist das Hauptprodukt bei allen Bedingungen. Die dargestellten Reaktionsmechanismen der Bildung von Acetaldehyd sind hier unterschiedlich zu den bis dahin angenommenen Reaktionswegen. Das komplette Reaktionsmodell und die kinetischen Parameter der Umsetzung von Glycerol können nach Optimierung an die erhaltenen Messdaten angepasst werden.

Es ist weiterhin bekannt, dass Acetaldehyd bei der homogenkatalysierten Dehydratisierung von Ethylenglykol in nah- und überkritischem Wasser entsteht [Ott2005]. So kann man Acetaldehyd mit einer Ausbeute von 10 % unter Zusatz katalytischer Mengen an Zinksulfat zu einer verdünnten Ethylenglykol-Lösung erhalten. Durch Verwendung von 20 mM Schwefelsäure als Katalysator kann die Ausbeute auf etwa 80 % bei 400 °C, 34 MPa und 15 s Verweilzeit erhöht werden. Darüber hinaus katalysiert Zinksulfat die Folgereaktionen von Acrolein aus der Dehydratisierung von Glycerol. Für eine wässrige 1 % (g g⁻¹) Acrolein-Lösung beträgt der Umsatz 62 % bei 360 °C, 34 MPa und 120 s Verweilzeit. Flüssige Reaktionsprodukte können nicht gefunden werden. Nachteilig ist wiederum die Korrosivität der Schwefelsäure unter den gegebenen Bedingungen.

Darüber hinaus ist bekannt, dass man Acetaldehyd bei der Dehydratisierung von sehr verdünnten wässrigen Glycerol-Lösungen ohne Zusatz oder Zugabe von Schwefelsäure unter nah- und überkritischen Bedingungen im Batch- oder Strömungsrohr-Reaktor erhält [Wat2007]. Die maximale Ausbeute an Acetaldehyd beträgt etwa 23 % für die kontinuierliche Dehydratisierung einer 0,05 M Glycerol-Lösung bei 400 °C, 34,5 MPa, 20 s Verweilzeit und Zugabe von 5 mM Schwefelsäure. Die Ausbeuten ohne Katalysator sind bedeutend geringer. Nachteilig sind die geringen Ausgangskonzentrationen von Glycerol bei gleichzeitiger Verwendung von Schwefelsäure als Katalysator.

Das zu lösende technische Problem besteht darin, das produzierte Acrolein in einer zweiten Stufe kontinuierlich in hohen Ausbeuten ohne Einsatz von Schwefelsäure und mit kurzen Verweilzeiten zu Acetaldehyd umzusetzen, um die Gesamtausbeute an Acetaldehyd aus Glycerol zu erhöhen. Dieses Problem wird durch das erfindungsgemäße Verfahren, welches **dadurch gekennzeichnet** ist, dass Acrolein und ein oder mehrere in Wasser gelöste Ammoniumsalze unter hohen Drücken und Temperaturen von 300-400 °C kontinuierlich umgesetzt werden, gelöst.

Das erfindungsgemäße Verfahren läuft bevorzugt in einem pH-Bereich von 4-8, besonders bevorzugt von 4-6 ab.

Es ist erfindungsgemäß besonders bevorzugt, die Umsetzung im sauren Reaktionsmilieu durchzuführen, wodurch die Bildung von Metallhydroxiden und/oder Polymerisationsreaktionen des Acroleins verhindert werden kann.

Besonders bevorzugt sind anorganische Ammoniumsalze, insbesondere Ammoniumsulfat, Ammoniumhydrogensulfat, Ammoniumacetat und Ammoniumdihydrogenphosphat.

Dabei führt der Einsatz der Ammoniumsalze zu einer Einstellung eines pH-Bereiches, in dem die Retro-Aldol-Reaktion vom Acrolein zu Acetaldehyd begünstigt wird. Neben Acetaldehyd werden 3-Methylpyridin und gasförmige Produkte gebildet. Zusätzlich entsteht als Koppelprodukt Formaldehyd, was qualitativ nachgewiesen werden konnte.

Die Separation des Acetaldehyds von 3-Methylpyridin kann mit sehr geringem Aufwand erfolgen.

Das erfindungsgemäße Verfahren erreicht eine maximale Ausbeute an Acetaldehyd von 40-62 % bezogen auf die eingesetzten Ausgangsverbindungen.

Das erfindungsgemäße Verfahren kann sowohl direkt mit dem Acrolein enthaltenden Reaktionsgemisch des Acrolein-Syntheseschrittes als auch mit vorher gereinigtem Acrolein durchgeführt werden.

Abhängig von der Dichte des Mediums werden erfindungsgemäß bevorzugt Verweilzeiten von 5-240s eingestellt.

Die Umsetzungen erfolgen erfindungsgemäß bevorzugt bei maximal 400°C und 40 MPa.

Das erfindungsgemäße Verfahren kann in standardmäßigen Hochdruckanlagen durchgeführt werden. Bevorzugt wird hierbei eine Anlage mit einem Strömungsrohrreaktor aus Inconel625 und einem Reaktorvolumen von 4-50 ml. Die Ausgangsmischungen werden über zwei vorgeheizte seperate Stränge mit maximal 35 ml min⁻¹ in den Reaktor gefördert.

Die vorliegende Erfindung wird durch die folgenden, nicht limitierenden Beispiele näher erläutert.

### Beispiel 1

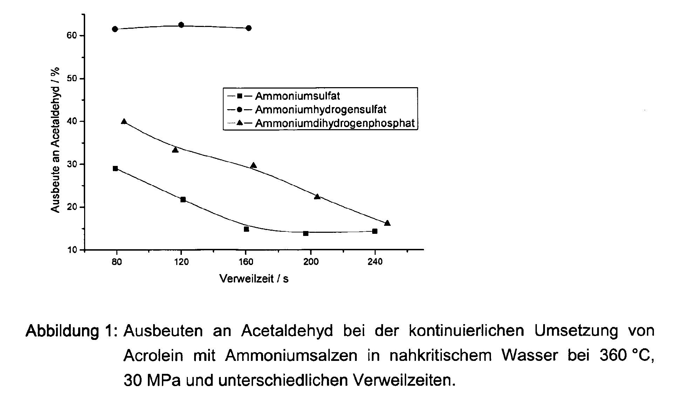

Eine wässrige Lösung mit 0,75 % (g g⁻¹) Acrolein und 1,77 % (g g⁻¹) Ammoniumsulfat oder 3,15 % (g g⁻¹) Ammoniumhydrogensulfat oder 3,07 % (g g⁻¹) Ammoniumdihydrogenphosphat, was einem molaren Verhältnis von Acrolein zu Ammoniumionen von 1:2 entspricht, wird in einer zweisträngigen Hochdruckanlage bei 30 MPa umgesetzt. Das flüssige Gemisch wird zunächst in einer Vorheizung auf 170 °C erwärmt und anschließend mit der doppelten Menge an heißem Wasser gemischt, so dass am Reaktoreingang eines Rohrreaktor aus Inconel625 und Volumens von 49,5 ml, die Reaktionstemperatur von 360 °C eingestellt wird und nahkritische Bedingungen des Wassers herrschen. Je nach Volumenfluss und Dichte des Reaktionsmediums werden Verweilzeiten von 60-240 s eingestellt. Die Reaktionslösung wird anschließend in einem Wärmetauscher auf Raumtemperatur abgekühlt und auf Atmosphärendruck entspannt. In einem Phasenseperator werden bei 2 °C die flüssigen Komponenten von den gasförmigen getrennt. Die flüssige Phase wird gesammelt und die Anteile der nachweisbaren Komponenten gaschromatographisch bestimmt. Zur quantitativen Bestimmung des Acetaldehyds und des Acroleins wird die Probe mit 1-Butanol als interner Standard versetzt. Die ermittelten Ausbeuten von Acetaldehyd unter den beschriebenen Bedingungen sind in Abbildung 1 dargestellt. Die maximale Ausbeute an Acetaldehyd beträgt 62 % bei allen gemessenen Verweilzeiten mit Ammoniumhydrogensulfat.

### Beispiel 2

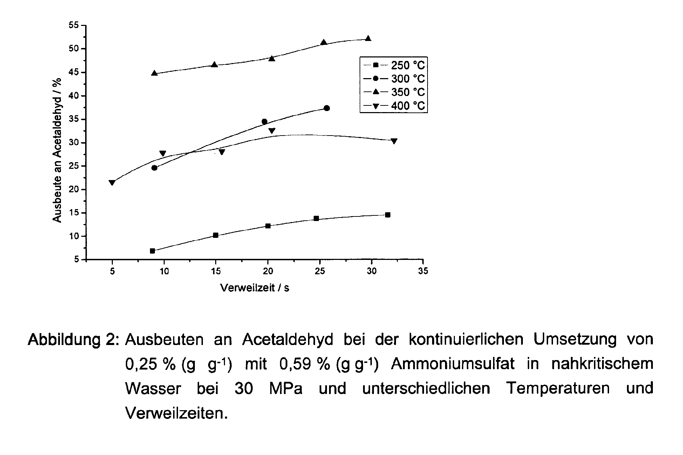

Die Umsetzung erfolgt mit Acrolein und Ammoniumsulfat in einem molaren Verhältnis von 1:1. Eine wässrige Lösung mit 0,75 % (g g⁻¹) Acrolein wird zunächst in einer Vorheizung auf 50 °C erwärmt und anschließend mit der doppelten Menge einer vorgewärmten wässrigen Lösung mit 0,89 % (g g⁻¹) Ammoniumsulfat gemischt, so dass die Reaktionstemperatur am Reaktoreingang eines Strömungsrohrreaktors (Inconel625; 4,4 ml Reaktorvolumen) eingestellt wird. Je nach Volumenfluss und Dichte des Reaktionsmediums werden Verweilzeiten von 5-35 s eingestellt. Die Ergebnisse sind in Abbildung 2 dargestellt. Die maximale Ausbeute an Acrolein beträgt 52 % bei einer Temperatur von 350 °C und einer Verweilzeit von 30 s.

### Literatur

- [Ant1985]: M. J. Antal Jr., W. S. L. Mok, J. C. Roy, A. C. Raissi, D. G. M. Anderson: Pyrolytic sources of hydrocarbons from Biomass, Journal of Analytical and Applied Pyrolysis, 1985, 8, 291-303.
- [Ant1987]: M. J. Antal Jr., A. Britain, C. DeAlmeida, W. S. L. Mok, S. Ramayya: Catalyzed and uncatalyzed conversion of cellulose biopolymer model compounds to chemical feedstocks in supercritical solvents, Energy from Biomass and Wastes, 1987, 10, 865-877.
- [Büh2002]: W. Bühler, E. Dinjus, H. J. Ederer, A. Kruse, C. Mas: lonic reactions and pyrolysis of glycerol as competing reaction pathways in near- and supercritical water, JournalofSupercriticalFluids, 2002, 22, 37-53.
- [Eck2007]: M. Eckert, G. Fleischmann, R. Jira, H. M. Bolt, K. Golka: Acetaldehyde, Ullmann's Encyclopedia of Industrial Chemistry, 7. Aufl., Wiley Interscience, Online Release, 2009.
- [Ott2005]: L. Ott: Stoffliche Nutzung von Biomasse mit Hilfe von nah- und überkritischem Wasser- homogenkatalysierte Dehydratisierung von Polyolen zu Aldehyden -, Dissertation, TU Darmstadt, 2005.
- [Ott2006]: L. Ott, M. Bicker, H. Vogel: Catalytic dehydration of glycerol in sub- and supercritical water: a new chemical process for acrolein production, Green Chemistry, 2006, 8, 214-220.
- [Ram1987]: S. Ramayya, A. Brittain, C. DeAlmeida, W. S. L. Mok, M. J. Antal Jr.: Acid-catalyzed dehydration of alcohols in supercritical water, Fuel, 1987, 66(10), 1364-71.
- [Wat2007]: M. Watanabe, T. lida , Y. Aizawa, T. M. Aida, H. Inomata: Acrolein synthesis from glycerol in hot-compressed water, Bioresource Technolgy, 2007, 98, 1285-1290.

## Patentansprüche

1. Verfahren zur selektiven Herstellung von Acetaldehyd, **dadurch gekennzeichnet, dass** Acrolein und ein oder mehrere in Wasser gelöste Ammoniumsalze unter hohen Drücken und Temperaturen von 300-400 °C kontinuierlich umgesetzt werden.

2. Verfahren gemäß Anspruch 1, **dadurch gekennzeichnet, dass** Ammoniumsulfat, Ammoniumhydrogensulfat, Ammoniumacetat und/oder Ammonium-dihydrogenphosphat eingesetzt werden.

3. Verfahren gemäß mindestens einem der Ansprüche 1-2, **dadurch gekennzeichnet, dass** das Acrolein und das Ammoniumsalz in einem Molverhältnis von 1:0,125 bis 1:2 eingesetzt werden.

4. Verfahren gemäß mindestens einem der Ansprüche 1-3, **dadurch gekennzeichnet, dass** die Umsetzung bei Drücken von 20-40 MPa erfolgt.

5. Verfahren gemäß mindestens einem der Ansprüche 1-4, **dadurch gekennzeichnet, dass** die Kontakt- bzw. Verweilzeit 5-240 s, bevorzugt 30-160 s beträgt.

6. Verfahren gemäß mindestens einem der Ansprüche 1-5, wobei der pH-Wert der wässrigen Lösung in einem Bereich von 4-8 liegt.

7. Verfahren gemäß mindestens einem der Ansprüche 1-6, wobei die Ausbeute an Acetaldehyd 40-62 % bezogen auf die eingesetzten Ausgangsverbindungen beträgt.

8. Verfahren gemäß mindestens einem der Ansprüche 1-7, wobei als Koppelprodukt Formaldehyd entsteht.

9. Verfahren gemäß mindestens einem der Ansprüche 1-8, wobei das Acrolein aus Glycerin gewonnen wird.
